# FASCICULE DE BREVET EUROPEEN

(11) **EP 1 593 372 B1**
(45) Date de publication et mention de la délivrance du brevet: **03.10.2007**
(21) Numéro de dépôt: 05290874.6
(22) Date de dépôt: 20.04.2005
(51) Int. Cl.: A61K 8/26, A61K 8/73, A61Q 19/08, A61Q 19/10

(54) **Composition cosmétique comprenant des particules de corindon et un polyholoside hétérogène**
Kosmetische Zusammensetzung enthaltend Korundpartikel und ein Hetero-polysaccharid
Cosmetic composition containing corundum particles and a hetero-polysaccharide

(30) Priorité: 07.05.2004 FR 0450881
(43) Date de publication de la demande: 09.11.2005
(73) Titulaire: L'ORÉAL, 75008 Paris (FR)
(72) Inventeur: Touzan, Philippe, 75012 Paris (FR)
(74) Mandataire: Renard, Emmanuelle

(56) Documents cités:
- EP-A- 0 045 493
- EP-A- 0 818 200
- WO-A-02/072042
- US-A- 2 996 432
- US-B1- 6 290 976
- DATABASE CAPLUS [Online] CHEMICAL ABSTRACTS SERVICE, COLUMBUS, OHIO, US; 3 avril 1989 (1989-04-03), MIYAMA, TAKASHI: "Cosmetic compositions containing mineral components" XP002310731 extrait de STN Database accession no. 1989:121056 & JP 63 039808 A2 (LISBURN PRODUCTS K. K., JAPAN) 20 février 1988 (1988-02-20)
- DATABASE CAPLUS [Online] CHEMICAL ABSTRACTS SERVICE, COLUMBUS, OHIO, US; 28 mars 2003 (2003-03-28), YAMADA, TAKASHI: "Marshmallow-touch cosmetic packs containing starch-acrylic acid graft copolymers" XP002310732 extrait de STN Database accession no. 2003:239795 & JP 2003 089615 A2 (POLA CHEMICAL INDUSTRIES, INC., JAPAN) 28 mars 2003 (2003-03-28)
- DATABASE CAPLUS [Online] CHEMICAL ABSTRACTS SERVICE, COLUMBUS, OHIO, US; 29 août 2002 (2002-08-29), YAMADA, TAKASHI: "Alginate-based cosmetic pack kits" XP002310733 extrait de STN Database accession no. 2002:654342 & JP 2002 241220 A2 (POLA CHEMICAL INDUSTRIES, INC., JAPAN) 28 août 2002 (2002-08-28)

## Description

La présente invention se rapporte à une composition cosmétique renfermant, dans un milieu physiologiquement acceptable, au moins un polyholoside hétérogène et des particules de corindon.

Elle concerne également un procédé cosmétique de soin de la peau ou procédé de "microdermabrasion", pour atténuer les irrégularités visibles ou tactiles de la surface de la peau, en particulier pour atténuer les rides et ridules et/ou les taches cutanées et/ou l'apparence des points noirs et/ou les pores de la peau et/ou lisser la peau et/ou unifier le teint, comprenant les étapes suivantes :
- l'application topique par massage sur la peau préalablement mouillée de la composition précitée, et
- le rinçage à l'eau de la composition.

La peau humaine est constituée de deux compartiments à savoir un compartiment superficiel, l'épiderme, et un compartiment profond, le derme.

L'épiderme humain naturel est composé principalement de trois types de cellules qui sont les kératinocytes, très majoritaires, les mélanocytes et les cellules de Langerhans. Chacun de ces types cellulaires contribue par ses fonctions propres au rôle essentiel joué dans l'organisme par la peau.

Au cours du processus dit de kératinisation, les kératinocytes situés dans la couche basale de l'épiderme se multiplient et croissent, poussant ainsi les cellules épidermiques plus anciennes vers le haut et vers la surface de l'épiderme. Lors de ce déplacement, ces cellules s'aplatissent et se différencient pour former de la kératine. Les cellules mortes superficielles résultant de ce processus de kératinisation (cornéocytes) constituent la couche cornée de l'épiderme où elles sont séparées par des couches lipidiques et reliées entre elles par des liaisons protéiniques (cornéosomes). Ces cellules mortes sont progressivement éliminées de la surface de la peau et remplacées par de nouvelles cellules kératinisées.

Dans la peau jeune et saine, la desquamation de la peau qui se produit ainsi est caractérisée par l'élimination de cellules individuelles ou de petits amas cellulaires. Au contraire, avec l'âge ou dans le cas de certaines pathologies, la desquamation peut être altérée, en ce sens qu'un excès de matière kératinique se forme à la surface de la peau, résultant soit en une élimination du stratum corneum sous forme de squames (vieillissement cutané, peau sèche), soit en une obstruction des follicules sébacés (acné).

On connaît dans l'art antérieur divers agents destinés à favoriser la desquamation, c'est-à-dire l'élimination des cellules « mortes » situées à la surface de la couche cornée de l'épiderme. Cette propriété "desquamante" est aussi appelée, souvent à tort, propriété kératolytique.

Ainsi, le brevet US 4,603,146 décrit l'emploi d'acide rétinoïque et de ses dérivés dans des compositions cosmétiques, en vue de lutter contre le vieillissement cutané. Par ailleurs, de nombreux brevets et publications (voir par exemple la demande EP-A-413 528) ainsi que de nombreuses compositions cosmétiques du commerce enseignent l'emploi des α-hydroxyacides comme l'acide lactique, l'acide glycolique ou encore l'acide citrique pour traiter le vieillissement cutané. On connaît enfin les β-hydroxyacides et plus spécialement l'acide salicylique ainsi que ses dérivés pour leur propriétés desquamantes (voir les documents WO-A-93/10756 et US 4,767,750).

A côté de ces desquamants "chimiques", il est connu d'utiliser des abrasifs tels que l'oxyde d'aluminium sous forme de corindon pour exfolier la surface de la peau (US-6,290,976 ; WO 02/072042). Ce procédé d'exfoliation est connu sous le terme de "microdermabrasion". La microdermabrasion est notamment mise en oeuvre par des esthéticiennes ou des dermatologues qui ont recours à des micro-cristaux d'oxyde d'aluminium (sous forme de corindon) pour resurfacer les couches superficielles de la peau. Ces cristaux sont projetés sur la peau au moyen d'un appareil qui les aspire ensuite avec la peau abrasée.

Bien que leur efficacité soit satisfaisante, tous ces composés desquamants et abrasifs de l'art antérieur peuvent générer des phénomènes d'irritation ou d'inconfort, en particulier chez les sujets à peau sensible.

En particulier, les abrasifs sont généralement choisis d'une taille suffisante pour ne pas obstruer les pores de la peau et risquer d'y produire une infection ou une inflammation, mais les abrasifs de taille supérieure à 100 µm ont généralement tendance à être plus irritants pour la peau que ceux de petite taille.

II subsiste donc le besoin de disposer d'une composition abrasive qui soit bien tolérée tout en étant efficace.

Or, la Demanderesse a maintenant découvert qu'en associant certains polyholosides à des particules de corindon, il était possible de masquer le caractère dur et coupant de cet oxyde métallique et d'obtenir ainsi une composition présentant une tolérance et une confort cutanés acceptables, tout en conservant une bonne efficacité abrasive. Il devient ainsi possible de formuler des compositions qui peuvent être utilisées à domicile, sans risque pour l'utilisatrice, ou qui peuvent être utilisées en institut sans nécessiter d'appareillage particulier.

Certains de ces polyholosides, en particulier les alginates, ont déjà été utilisés dans des masques (US-2,996,432 ; EP-0 045 493 ; JP-2003 089615 ; JP-2002 241220) en raison de leur capacité à former un gel rigide en présence de sels métalliques. Dans la demande EP-0 045 493, il est suggéré de les associer notamment à de l'oxyde d'aluminium. Il s'agit toutefois d'un composé utilisé pour modifier la texture ou la stabilité du gel formé par l'alginate, donc très vraisemblablement d'oxyde d'aluminium sous forme d'alumine amorphe. L'alumine amorphe se forme vers 50-60°C et est couramment utilisée comme charge incolore lorsqu'elle se trouve sous forme hydratée. Elle se distingue ainsi du corindon, obtenu généralement par électro-fusion d'alumine calcinée à haute température (plus de 350°C), et qui correspond à l'alumine alpha de forme hexagonale, utilisée couramment comme abrasif.

D'autres polyholosides, à base de fucose, ont par ailleurs été décrits comme agents desquamants (EP-0 818 200). Il n'a toutefois jamais été suggéré de les associer à des particules de corindon, en particulier pour une mise en oeuvre dans un procédé de microdermabrasion.

La présente invention a donc pour objet une composition comprenant, dans un milieu physiologiquement acceptable, au moins un polyholoside hétérogène et des particules de corindon.

Le polyholoside a l'avantage non seulement de réduire l'irritation résultant du caractère abrasif du corindon, mais également de compléter cette action abrasive, mécanique, par une action exfoliante "biologique", en induisant la libération de cornéocytes, comme démontré dans la demande EP-0 818 200.

Ces deux constituants de la composition selon l'invention seront maintenant décrits plus en détail.

### Polyholoside hétérogène

Par "polyholoside hétérogène", on entend selon la présente invention des polymères constitués de l'association d'oses différents ou d'oses ayant la même formule chimique brute mais de configuration géométrique différente (isomères D et L par exemple).

Ces polymères se distinguent à la fois des polyhétérosides, qui sont constitués d'un ou plusieurs oses et d'une partie non glucidique, et des polyholosides homogènes, qui résultent de l'association d'un même ose.

Ainsi, le polyholoside hétérogène selon l'invention est constitué uniquement d'oses et résulte de l'association d'au moins deux oses différents.

Les polyholosides selon l'invention peuvent être constitués de 2 à 10 oses, composés couramment appelés oligoholosides, ou de plus de 10 oses, composés couramment appelés polyholosides.

Les oses présents dans le polyholoside selon l'invention peuvent être choisis parmi tous les oses envisageables, d'origine naturelle ou synthétique, et notamment tels que :
- les aldoses comme
   . les pentoses : ribose, arabinose, xylose ou apiose, par exemple,
   . les hexoses : glucose, fucose, mannose ou galactose, par exemple,
- les cétoses tels que le fructose,
- les désoxyoses, tels que le rhamnose, le digitoxose, le cymarose ou l'oléandrose,
- les dérivés d'ose tels que les acides uroniques comme les acides mannuronique, guluronique, galacturonique ou glycuronique, ou encore les itols comme le mannitol ou le sorbitol.

Dans le cadre de la présente invention, on peut utiliser un polyholoside hétérogène seul, ou un mélange de polyholosides hétérogènes.

Le polyholoside selon l'invention peut être ramifié ou linéaire. Il peut également être substitué, par exemple par des chaînes grasses, notamment comprenant 8 à 30 atomes de carbone.

D'autre part, le polyholoside selon l'invention peut être un alginate (poly mannuronate et guluronate) tel qu'un alginate de sodium, un alginate de propylène glycol, un alginate de calcium, ou un alginate de glycéryle.

Toutefois, le polyholoside hétérogène comprend de préférence au moins un motif fucose, qui peut être présent en une quantité de 10-90% en poids, de préférence 15-35% en poids, par rapport au poids de matière sèche de polyholoside.

En particulier, le polyholoside selon l'invention peut comprendre des motifs fucose, galactose et acide galacturonique, et, par exemple peut comprendre un enchaînement linéaire de α-L-Fucose, de α-D-Galactose et d'acide galacturonique. Dans ce cas, il présente de préférence une viscosité de 800-1200 mPa.s (viscosité Brookfield LV31, 12 tours/min, à 30°C) lorsqu'il est mis en solution dans l'eau à une concentration d'environ 1% en poids. Un tel polyholoside est notamment disponible sous forme d'une solution à 1% dans l'eau auprès de la société SOLABIA sous la dénomination commerciale Fucogel 1000 PP®.

Les polyholosides selon l'invention sont de préférence introduits dans la composition sous forme de solution aqueuse qui peut comprendre 0,1 à 5% en poids de polyholoside.

Le polyholoside peut être présent dans la composition selon l'invention en une quantité représentant de 0,0001 à 1% en poids, de préférence de 0,0005 à 0,01% en poids, par rapport au poids total de la composition.

### Corindon

Le corindon utilisable selon l'invention est un oxyde d'aluminium, qui peut par exemple être enrobé, notamment d'un composé siliconé, mais est de préférence non enrobé.

Les particules de corindon présentent avantageusement une pureté d'au moins 95%, mieux, d'au moins 99%.

Leur taille moyenne de particule va de préférence de 100 à 180 µm.

Les particules de corindon utilisables selon l'invention sont en particulier des particules d'oxyde d'aluminium calcinées à haute température, jusqu'à obtenir la structure cristalline du corindon (α-Al₂O₃), puis traitées pour former des grains pourvus d'arêtes tranchantes et ayant une distribution de tailles de particules donnée, les particules ayant de préférence un diamètre de particule moyen compris entre 100 et 180 µm et de préférence entre 130 et 150 µm. Leur distribution est avantageusement telle qu'aucune des particules n'a un diamètre supérieur à 250 µm. De telles particules sont notamment disponibles dans le commerce auprès de la société MARKETECH INTERNATIONAL sous la dénomination commerciale Dermagrain. Les particules référencées Dermagrain 900 sont constituées d'alumine alpha cristallisée pure à 99,55%, présentant un diamètre moyen de particule d'environ 140 µm, les particules ayant toutes un diamètre inférieur à 250 µm. Moins de 3% des particules a un diamètre inférieur à 105 µm. D'autres particules sont disponibles auprès de la société INDUSTRIAL SUPPLY sous la dénomination commerciale ARL100 et ARL120. Il s'agit de particules d'oxyde d'aluminium ayant une taille moyenne de particule de 120 et 100 µm, respectivement, et une distribution de tailles de particules allant de 75 à 212 µm et de 63 à 180 µm, respectivement.

Les particules de corindon peuvent représenter de 5 à 40%, et de préférence de 10 à 30%, du poids total de la composition.

La composition selon l'invention est généralement adaptée à une application topique sur la peau et comprend donc généralement un milieu physiologiquement acceptable, c'est-à-dire compatible avec la peau et/ou ses phanères. Ainsi, la composition selon l'invention a de préférence un pH inférieur à 8, mieux, inférieur ou égal à 7 et, encore mieux, compris entre 5,5 et 7.

La composition selon l'invention peut se présenter sous toutes les formes galéniques classiquement utilisées pour une application topique, pour autant qu'elle soit rinçable à l'eau, et notamment sous forme de dispersions du type lotion ou gel ou d'émulsions de consistance liquide ou semi-liquide du type lait, obtenues par dispersion d'une phase grasse dans une phase aqueuse (H/E), ou de suspensions ou émulsions de consistance molle, semi-solide ou solide du type crème ou gel, obtenues par dispersion d'une phase grasse dans une phase aqueuse (H/E), ou encore d'émulsions multiples (E/H/E), de microémulsions, de dispersions vésiculaires de type ionique et/ou non ionique, ou de dispersions cire/phase aqueuse. Ces compositions sont préparées selon les méthodes usuelles.

Selon un mode préféré de réalisation de l'invention, la composition se présente sous forme d'une émulsion huile-dans-eau (H/E).

Comme huiles utilisables dans la composition selon l'invention, on peut citer :
- les huiles hydrocarbonées d'origine animale, telles que le perhydrosqualène ;
- les huiles hydrocarbonées d'origine végétale, telles que les huiles végétales, en particulier la fraction liquide du beurre de karité;
- les esters et les éthers de synthèse, notamment d'acides gras, comme par exemple les esters du pentaérythritol tels que le tétraéthylhexanoate de pentaérythrityle ;
- les hydrocarbures linéaires ou ramifiés, d'origine minérale ou synthétique, tels que le polyisobutène hydrogéné et l'huile minérale ;
- les alcools gras ayant de 8 à 26 atomes de carbone, comme l'alcool cétylique, l'alcool stéarylique et leur mélange, ou l'octyldodécanol ;
- les huiles de silicone comme les polyméthylsiloxanes (PDMS) volatiles ou non à chaîne siliconée linéaire ou cyclique ; et
- leurs mélanges.

Lorsqu'elle est présente, la phase grasse de la composition selon l'invention peut comprendre, outre des huiles, d'autres corps gras tels que : les acides gras comportant de 8 à 30 atomes de carbone ; les cires ; et les gommes telles que les gommes de silicone (diméthiconol).

Ces corps gras peuvent être choisis de manière variée par l'homme du métier afin de préparer une composition ayant les propriétés, par exemple de consistance ou de texture, souhaitées.

Cette composition peut en outre contenir divers adjuvants couramment utilisés dans le domaine cosmétique, tels que des émulsionnants dont le stéarate de glycéryle et les stéarates de poly(oxyde d'éthylène) ; des charges ; des conservateurs ; des séquestrants ; des colorants ; des parfums ; et des épaississants et gélifiants, en particulier les homo- et copolymères d'acrylamide et les homo- et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société CLARIANT sous la dénomination « Hostacerin AMPS ». Les quantités de ces différents adjuvants et leur nature seront choisis de manière à ne pas nuire aux propriétés de la composition selon l'invention.

Pour renforcer les effets de la composition selon l'invention, celle-ci peut renfermer par ailleurs au moins un composé choisi parmi : les agents hydratants tels que les céramides, la glycérine et l'acide hyaluronique ; les agents desquamants tels que les α-hydroxyacides, dont les acides glycolique et lactique, les β-hydroxyacides, en particulier l'acide n-octanoyl-5-salicylique, l'HEPES ou le miel ; les agents anti-séborrhéiques tels que les sels de zinc ou certains extraits de Laminaires ou d'ulmaire ; les agents apaisants tels que l'acide β-glycyrrhétinique et ses sels et/ou ses dérivés et les extraits de pivoine ou d'aloe vera ; et leurs mélanges.

La composition selon l'invention peut être appliquée sur la peau par massage manuel du bout des doigts, ou par massage mécanique au moyen d'un appareil vibrant pourvu d'une tête de massage munie d'un tampon, tel que décrit dans la demande US 2001/0046506 ou le brevet US-6,652,888, par exemple.

La composition selon l'invention permet d'estomper les irrégularités visibles et tactiles de la surface cutanée, en raison de sa capacité à abraser mécaniquement la surface de la peau, mais également à accélérer le renouvellement cellulaire.

L'invention a donc aussi pour objet un procédé cosmétique de soin de la peau ou procédé de "microdermabrasion", pour atténuer les irrégularités visibles ou tactiles de la surface de la peau, en particulier pour atténuer les rides et ridules et/ou les taches cutanées et/ou l'apparence des points noirs et/ou les pores de la peau et/ou lisser la peau et/ou unifier le teint, comprenant les étapes suivantes :
- l'application topique par massage sur la peau préalablement mouillée de la composition précitée, et
- le rinçage à l'eau de la composition.

La fréquence à laquelle ce procédé peut être mis en oeuvre dépendra du résultat recherché. Il sera généralement mis en oeuvre une à trois fois par semaine, de préférence deux fois par semaine, pendant une durée allant de une semaine à six mois, de préférence de quatre à huit semaines.

En outre, le procédé selon l'invention peut comprendre une étape supplémentaire d'application sur la peau d'une composition hydratante et/ou apaisante.

Ce procédé est avantageusement mis en oeuvre sur des personnes présentant des signes de vieillissement cutané, tels que des rides et ridules et/ou des irrégularités de pigmentation et/ou un teint terne, ou sur des personnes présentant des pores dilatés et/ou des points noirs.

L'invention sera maintenant illustrée par les exemples non limitatifs suivants. Dans ces exemples, les quantités sont indiquées en pourcentage pondéral.

### EXEMPLES

### Exemple 1 : Crème de microdermabrasion

On prépare une émulsion H/E ayant la composition ci-dessous, de façon classique pour l'homme du métier.

| | | |
|---|---|---|
| *Phase A* | Stéarate de PEG (100 OE) | 0,75% |
| | Stéarate de glycéryle | 0,75 % |
| | Alcool stéarylique | 1,5 % |
| | Huile minérale | 10 % |
| | Huile d'amande d'abricots | 2 % |
| | Polydiméthylsiloxane | 1 % |
| | | |
| *Phase B* | Eau déminéralisée | qsp 100 % |
| | Polyholoside à 1 %* | 1 % |
| | Polyacrylamide | 0,3 % |
| | Butylène glycol | 3 % |
| | Propylène glycol | 3 % |
| | | |
| *Phase C* | Corindon** | 15 % |
| | | |
| | Conservateurs | qs |
| | | |

| | | |
|---|---|---|
| * Fucogel 1000 PP de SOLABIA ** Dermagrain 900 de MarkeTech International | | |

Cette crème peut être appliquée le soir sur le visage mouillé, en évitant le contour des yeux et des lèvres, par massage du bout des doigts pendant environ 1 minute 30, suivi d'un rinçage à l'eau. Elle permet d'améliorer l'éclat du teint, de lisser sa surface et de réduire l'apparence des rides.

### Exemple 2 : Gel moussant désincrustant

On prépare la composition suivante de façon classique pour l'homme du métier :

| | | |
|---|---|---|
| *Phase A* | Eau déminéralisée | qsp 100 % |
| | EDTA | 0,2 % |
| | Conservateur | qs |
| | Copolymère acrylique (PEMULEN TR1) | 1 % |
| | Sodium lauroyl sarcosinate | 2 % |
| | Sodium laureth sulfate | 5 % |
| | | |
| *Phase B* | Eau déminéralisée | 10 % |
| | Polyholoside à 1 %* | 2 % |
| | Gomme de xanthane | 0,3 % |
| | Butylène glycol | 3 % |
| | Propylène glycol | 3 % |
| | Conservateur | qs |
| | Triéthanolamine | 0,8 % |
| | Corindon** | 6 % |

| | | |
|---|---|---|
| * Fucogel 1000 PP de SOLABIA ** Dermagrain 900 de MarkeTech International | | |

Ce gel peut être appliquée le matin ou le soir sur le visage mouillé, en évitant le contour des yeux et des lèvres, par massage du bout des doigts pendant environ 1 minute 30, suivi d'un rinçage à l'eau. Il permet de réduire l'apparence des points noirs et de resserrer les pores de la peau.

### Exemple 3 : Test in vivo

### Principe :

On a testé en hémi-visage sur un panel de 10 personnes deux compositions A et B, constituées chacune d'une émulsion huile-dans-eau gélifiée renfermant 20% en poids de particules de corindon (Dermagrain 900 de MarkeTech International), la composition B contenant en plus 0,2% en poids de polysaccharide hétérogène à 1% en poids dans l'eau (Fucogel 1000 PP de SOLABIA).

### Protocole :

Après humidification du visage à l'éponge, l'esthéticienne a appliqué 1 g de produit par demi-visage, suivi d'un léger massage puis de l'élimination du produit par rinçage à l'éponge. Le visage a ensuite été séché par tapotements à l'aide d'un mouchoir.

### Résultat:

Pour 4 sujets sur 10, la composition A est perçue comme plus abrasive que la composition B, en ce sens que les grains semblent plus gros, plus durs, plus perceptibles que sur l'autre moitié du visage traité à l'aide de la composition B. En outre, 7 des 10 sujets ont interrompu l'application de la composition A en raison de ces inconforts, jugeant la composition B plus confortable, alors que 3 sujets sur 10 seulement ont interrompu l'application de la composition B.

En outre, après application des compositions A et B, la peau paraît fraîche, propre, plus douce et plus lisse qu'avant l'application.

Ces résultats montrent donc que la composition B est perçue comme aussi efficace mais nettement moins abrasive que la composition A, et donc mieux tolérée.

## Revendications

1. Composition cosmétique renfermant, dans un milieu physiologiquement acceptable, au moins un polyholoside hétérogène et des particules de corindon.

2. Composition selon la revendication 1, **caractérisée en ce que** ledit polyholoside hétérogène est un alginate.

3. Composition selon la revendication 1, **caractérisée en ce que** ledit polyholoside hétérogène comprend au moins un motif fucose.

4. Composition selon la revendication 3, **caractérisée en ce que** ledit polyholoside hétérogène comprend des motifs fucose, galactose et acide galacturonique, en particulier un enchaînement linéaire de α-L-Fucose, de α-D-Galactose et d'acide galacturonique.

5. Composition selon l'une quelconque des revendications 1 à 4, **caractérisée en ce que** ledit polyholoside représente de 0,0005 à 0,01 % en poids, par rapport au poids total de la composition.

6. Composition selon l'une quelconque des revendications 1 à 5, **caractérisée en ce que** la taille moyenne des particules de corindon va de 100 à 180 µm.

7. Composition selon la revendication 6, **caractérisée en ce que** les particules de corindon ont un diamètre de particule moyen compris entre 130 et 150 µm.

8. Composition selon la revendication 6 ou 7, **caractérisée en ce qu**'aucune des particules de corindon n'a un diamètre supérieur à 250 µm.

9. Composition selon l'une quelconque des revendications 1 à 8, **caractérisée en ce que** les particules de corindon représentent de 5 à 40% du poids total de la composition.

10. Composition selon la revendication 9, **caractérisée en ce que** les particules de corindon représentent de 10 à 30% du poids total de la composition.

11. Composition selon l'une quelconque des revendications 1 à 10, **caractérisée en ce qu**'elle a un pH compris entre 5,5 et 7.

12. Composition selon l'une quelconque des revendications 1 à 11 **caractérisée en ce qu'**elle se présente sous forme d'une émulsion huile-dans-eau.

13. Procédé cosmétique de soin de la peau, destiné à atténuer les irrégularités visibles ou tactiles de la surface de la peau, comprenant les étapes suivantes :
- l'application topique par massage sur la peau préalablement mouillée de la composition selon l'une quelconque des revendications 1 à 12 et
- le rinçage à l'eau de ladite composition.

14. Procédé selon la revendication 13, **caractérisé en ce qu'**il est destiné à atténuer les rides et ridules et/ou les taches cutanées et/ou l'apparence des points noirs et/ou les pores de la peau et/ou à lisser la peau et/ou à unifier le teint.

15. Procédé selon la revendication 13 ou 14, **caractérisé en ce qu**'il est mis en oeuvre une à trois fois par semaine.

16. Procédé selon l'une quelconque des revendications 13 à 15, **caractérisé en ce qu**'il est mis en oeuvre sur des personnes présentant des signes de vieillissement cutané, tels que des rides et ridules et/ou des irrégularités de pigmentation et/ou un teint terne, ou sur des personnes présentant des pores dilatés et/ou des points noirs.

## Claims

1. Cosmetic composition containing, in a physiologically acceptable medium, at least one heterogeneous polyholoside and corundum particles.

2. Composition according to Claim 1, **characterized in that** the said heterogeneous polyholoside is an alginate.

3. Composition according to Claim 1, **characterized in that** the said heterogeneous polyholoside comprises at least one fucose unit.

4. Composition according to Claim 3, **characterized in that** the said heterogeneous polyholoside comprises fucose, galactose and galacturonic acid units, in particular a linear sequence of α-L-fucose, α-D-galactose and galacturonic acid.

5. Composition according to any one of Claims 1 to 4, **characterized in that** the said polyholoside represents from 0.0005% to 0.01% by weight relative to the total weight of the composition.

6. Composition according to any one of Claims 1 to 5, **characterized in that** the mean size of the corundum particles ranges from 100 to 180 µm.

7. Composition according to Claim 6, **characterized in that** the corundum particles have a mean particle diameter of between 130 and 150 µm.

8. Composition according to Claim 6 or 7, **characterized in that** none of the corundum particles has a diameter of greater than 250 µm.

9. Composition according to any one of Claims 1 to 8, **characterized in that** the corundum particles represent from 5% to 40% of the total weight of the composition.

10. Composition according to Claim 9, **characterized in that** the corundum particles represent from 10% to 30% of the total weight of the composition.

11. Composition according to any one of Claims 1 to 10, **characterized in that** it has a pH of between 5.5 and 7.

12. Composition according to any one of Claims 1 to 11, **characterized in that** it is in the form of an oil-in-water emulsion.

13. Cosmetic skincare process for attenuating the visible or tactile irregularities on the surface of the skin, comprising the following steps:
- the topical application of the composition according to any one of Claims 1 to 12, by massaging into the premoistened skin, and
- rinsing of the said composition with water.

14. Process according to Claim 13, **characterized in that** it is intended for attenuating wrinkles and fine lines and/or skin marks and/or the appearance of blackheads and/or skin pores and/or for smoothing out the skin and/or for unifying the complexion.

15. Process according to Claim 13 or 14, **characterized in that** it is performed one to three times a week.

16. Process according to any one of Claims 13 to 15, **characterized in that** it is performed on individuals presenting signs of ageing of the skin, such as wrinkles and fine lines and/or pigmentation irregularities and/or a dull complexion, or on individuals with dilated pores and/or blackheads.

## Patentansprüche

1. Kosmetische Zusammensetzung, die in einem physiologisch annehmbaren Medium mindestens ein heterogenes Polysaccharid und Korundpartikel enthält.

2. Zusammensetzung nach Anspruch 1, **dadurch gekennzeichnet, dass** das heterogene Polysaccharid ein Alginat ist.

3. Zusammensetzung nach Anspruch 1, **dadurch gekennzeichnet, dass** das heterogene Polysaccharid zumindest eine Fucoseeinheit enthält.

4. Zusammensetzung nach Anspruch 3, **dadurch gekennzeichnet, dass** das heterogene Polysaccharid Fucose-, Galactose- und Galacturonsäureeinheiten, insbesondere eine lineare Verknüpfung von α-L-Fructose, α-D-Galactose und Galacturonsäure enthält.

5. Zusammensetzung nach einem der Ansprüche 1 bis 4, **dadurch gekennzeichnet, dass** das Polysaccharid 0,0005 bis 0,01 Gew.-%, bezogen auf das Gesamtgewicht der Zusammensetzung, ausmacht.

6. Zusammensetzung nach einem der Ansprüche 1 bis 5, **dadurch gekennzeichnet, dass** die mittlere Größe der Korundpartikel im Bereich von 100 bis 180 µm liegt.

7. Zusammensetzung nach Anspruch 6, **dadurch gekennzeichnet, dass** die Korundpartikel einen mittleren Teilchendurchmesser von 130 bis 150 µm besitzen.

8. Zusammensetzung nach Anspruch 6 oder 7, **dadurch gekennzeichnet, dass** kein Korundpartikel einen Durchmesser größer als 250 µm aufweist.

9. Zusammensetzung nach einem der Ansprüche 1 bis 8, **dadurch gekennzeichnet, dass** die Korundpartikel 5 bis 40 % des Gesamtgewichts der Zusammensetzung ausmachen.

10. Zusammensetzung nach Anspruch 9, **dadurch gekennzeichnet, dass** die Korundpartikel 10 bis 30 % des Gesamtgewichts der Zusammensetzung ausmachen.

11. Zusammensetzung nach einem der Ansprüche 1 bis 10, **dadurch gekennzeichnet, dass** sie einen pH-Wert von 5,5 bis 7 aufweist.

12. Zusammensetzung nach einem der Ansprüche 1 bis 11, **dadurch gekennzeichnet, dass** sie in Form einer Öl-in-Wasser-Emulsion vorliegt.

13. Kosmetisches Hautpflegeverfahren zur Verminderung sichtbarer oder spürbarer Unregelmäßigkeiten der Hautoberfläche, das folgende Schritte umfasst:
- topische Anwendung einer Zusammensetzung nach einem der Ansprüche 1 bis 12 auf die zuvor befeuchtete Haut durch Massieren, und
- das Abspülen der Zusammensetzung mit Wasser.

14. Verfahren nach Anspruch 13, **dadurch gekennzeichnet, dass** es dazu dient, Falten und Fältchen und/oder Hautflecken und/oder das Auftreten von Mitessern und/oder Hautporen zu vermindern und/oder die Haut zu glätten und/oder den Teint zu vereinheitlichen.

15. Verfahren nach Anspruch 13 oder 14, **dadurch gekennzeichnet, dass** es ein bis drei Mal pro Woche angewandt wird.

16. Verfahren nach einem der Ansprüche 13 bis 15, **dadurch gekennzeichnet, dass** es bei Personen angewendet wird, bei denen Zeichen der Hautalterung vorhanden sind, wie Falten und Fältchen und/oder Unregelmäßigkeiten der Pigmentierung und/oder ein glanzloser Teint, oder bei Personen, die erweiterte Poren und/oder Mitesser aufweisen.
